(19) Europäisches Patentamt
European Patent Office
Office européen des brevets

(11) **EP 3 501 382 A1**

(12) **EUROPEAN PATENT APPLICATION**

(43) Date of publication:
**26.06.2019 Bulletin 2019/26**

(51) Int Cl.:
***A61B 5/00*** *(2006.01)*   ***A61B 5/024*** *(2006.01)*

(21) Application number: **17210448.1**

(22) Date of filing: **22.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicants:
• **Stichting IMEC Nederland**
  **5656 AE  Eindhoven (NL)**
• **IMEC vzw**
  **3001 Leuven (BE)**

(72) Inventors:
• **ZHANG, Yifan**
  **3001 Leuven (BE)**
• **GROENENDAAL, Willemijn**
  **3001 Leuven (BE)**
• **SONG, Shuang**
  **3001 Leuven (BE)**
• **VULLINGS, Rik**
  **3001 Leuven (BE)**

(74) Representative: **AWA Sweden AB**
  **P.O. Box 5117**
  **200 71 Malmö (SE)**

(54) **A SYSTEM AND A METHOD FOR MOTION ARTIFACT REDUCTION IN A PPG SIGNAL**

(57)     A system (100) for motion artifact reduction in a photoplethysmogram, PPG, signal representing heart activity of a subject, comprises: a processing unit (120), said processing unit (120) being configured to: receive (302) a PPG signal carrying information of heart activity of the subject; receive (304) a motion reference signal carrying information of a motion of the subject; form (306) a PPG time-frequency representation, based on the PPG signal, and a motion reference time-frequency representation, based on the motion reference signal; and subtract (308) a weighted representation of the motion reference time-frequency representation from the PPG time-frequency representation for reducing a motion artifact component in the PPG time-frequency representation and form a cleaned time-varying frequency spectrum of the PPG signal.

Fig. 3

**Description**

<u>Technical field</u>

The present inventive concept relates to a system and a method for motion artifact reduction in a photoplethysmogram (PPG) signal. In particular, the inventive concept relates to reduction of motion artifacts due to periodic motions.

<u>Background</u>

Acquisition of signals representing a heart activity of a subject is important or of interest in many contexts. The signals may be used in clinical settings to provide information for treatment of the subject, but may also be used for general monitoring of a physical condition of the subject. Also, monitoring of heart activity may be of interest to the subject, e.g. for monitoring exercise or other activities of the subject. Photoplethysmography is a technology of interest in monitoring heart activity as it may be provided in a wearable device which may minimally affect a comfort of the subject wearing the device.

However, a photoplethysmogram (PPG) signal may be affected by motion artifacts. Thus, if the subject is moving, motion artifacts may be generated which may cause quality of an acquired PPG signal to deteriorate or may prevent making any assessments based on the PPG signal. This is accentuated, when the PPG signal is acquired during daily life of a subject. Thus, for long-term monitoring of heart activity using a PPG signal, motion artifact reduction is needed.

In Tăuţan A.-M., Young A., Wentink E., Wieringa F., "*Characterization and Reduction of Motion Artifacts in Photoplethysmographic Signals from a Wrist-worn Device*", Conference Proceedings: IEEE Engineering in Medicine and Biology Society 2015, pages 6146-9, methods for analyzing various motion artifacts in PPG signals, recorded by a wrist-worn device is disclosed. An analysis is made of the PPG signal properties in relation to possible motion reference signals, including X, Y, Z accelerometer axes, acceleration magnitude and a different wavelength PPG channel. The latter showed higher correlation to the input PPG signal during movement. A wavelet based motion artifact reduction algorithm is described. The algorithm improves the visibility of the heart rate components in the frequency domain representation of the signals. The proposed motion artifact reduction technique presented slight improvements in both time and frequency domain when compared to simply applying a bandpass filter. It is suggested that rhythmic motions like running or walking could be reduced through frequency eliminating techniques.

Thus, there is still a need of improvement in motion artifact reduction. Further, it would be advantageous to have a generic motion artifact reduction algorithm that does not need to identify and remove specific frequencies.

<u>Summary</u>

An objective of the present inventive concept is to provide an improved system and method for motion artifact reduction in a photoplethysmogram (PPG) signal.

This and other objectives of the invention are at least partly met by the invention as defined in the independent claims. Preferred embodiments are set out in the dependent claims.

According to a first aspect, there is provided a system for motion artifact reduction in a PPG signal representing heart activity of a subject, said system comprising: a processing unit, said processing unit being configured to: receive a PPG signal carrying information of heart activity of the subject; receive a motion reference signal carrying information of a motion of the subject; form a PPG time-frequency representation, based on the PPG signal, and a motion reference time-frequency representation, based on the motion reference signal; and subtract a weighted representation of the motion reference time-frequency representation from a weighted representation of the PPG time-frequency representation for reducing a motion artifact component in the PPG time-frequency representation and form a cleaned time-varying frequency spectrum of the PPG signal.

According to the invention, time-frequency representations are formed of the PPG signal and the motion reference signal. The motion reference signal may be more highly influenced by the motion artifact than the PPG signal. Thus, by subtracting the motion reference signal from the PPG signal, the influence of the motion artifact on the PPG signal may be reduced without need of e.g. completely removing signal in a frequency band where a motion artifact is present, which may also imply that heart activity information of the PPG signal is eliminated. This implies that influence of motion artifacts may be reduced without information of interest, e.g. heart activity information, in the PPG signal being unnecessarily removed from the PPG signal.

Hence, according to the invention, a robust technology for removing or at least reducing motion artifact influence on a PPG signal is provided.

If the PPG signal and motion reference signal are acquired in similar manner and/or based on sensors arranged closely to each other, the motion artifact may be represented in a similar way in the PPG signal and the motion reference signal. For such case, the subtraction of the motion reference signal from the PPG signal in time-frequency representation may be very effective in removing the motion artifact information from the PPG signal, since the motion artifact information may be represented in similar manner in the signals.

According to an embodiment, the motion reference signal may thus be acquired by a sensor arranged close to a sensor for acquiring the PPG signal. For instance, a PPG detector and a motion detector may be mounted in the system close to each other.

According to another embodiment, the motion reference signal may be acquired using a photoplethysmogram. The motion reference signal may thus be acquired using a wavelength which is relatively sensitive to motion artifacts, whereas the PPG signal may be acquired using a wavelength which is relatively insensitive to motion artifacts. This implies that the motion artifact information is emphasized in the motion reference signal, while the motion artifact information may be represented in a similar manner, as both the PPG signal and the motion reference signal are acquired using PPG technology. Hence, subtraction of the motion reference signal from the PPG signal in time-frequency representation may be very effective in removing the motion artifact information from the PPG signal.

According to an embodiment, the PPG signal is acquired using reflectance-type PPG, i.e. light is transmitted into tissue of the subject and diffusely reflected back towards a sensor. According to an embodiment, the motion reference signal may also be acquired using reflectance-type PPG.

The PPG signal may be acquired using a green or blue wavelength of light. For these wavelengths, especially in use of reflectance-type PPG, the PPG signal is relatively insensitive to motion artifacts as light penetrates a relatively small distance into tissue. The motion reference signal may be acquired using a red or infrared wavelength of light. For these wavelengths, especially in use of reflectance-type PPG, the PPG signal is relatively sensitive to motion artifacts as light penetrates a relatively large distance into tissue.

Hence, use of a green or blue wavelength in acquiring the PPG signal using reflectance-type PPG and use of a red or infrared wavelength in acquiring the motion reference signal using reflectance-type PPG may be particularly advantageous.

However, it should be realized that the PPG signal may be acquired in other ways, e.g. using transmittance-type PPG, and/or using different wavelengths of light. It should also be realized that the motion reference signal may be acquired in other ways, e.g. using transmittance-type PPG or even using other type of technology for detecting motions, such as an accelerometer. It should also be realized that any combination of technology for acquiring the PPG signal and technology for acquiring the motion reference signal may be contemplated.

As used herein, the term "time-frequency representation" should be construed as any representation that provides information of development of frequency content of a signal over time or information of time instances during which a frequency is present in the signal. Thus, in one embodiment, a spectrum of frequency content of the signal may be provided in each time instance (or short time interval). Hence, the "time-frequency representation" should be construed to provide both frequency information and information of variations over time for the signal.

Both the motion reference time-frequency representation and the PPG time-frequency representation may be weighted. The weighting of the representations may be performed to normalize each of the representations. The weighting of the motion reference time-frequency representation may be based on motion artifact information in the signals. Thus, the weighting of the motion reference time-frequency representation may ensure that relevant motion artifact information is represented by the PPG time-frequency representation and the motion reference time-frequency representation in a corresponding manner. This implies that subtraction of the weighted representation of the motion reference time-frequency representation from the weighted representation of the PPG time-frequency representation removes or at least reduces the motion artifact information in the PPG signal.

The cleaned time-varying frequency spectrum of the PPG signal may be used in order to directly extract information of heart activity, such as determining a heart rate of the subject. The cleaned time-varying frequency spectrum of the PPG signal may also or alternatively be used for reconstruction of a time domain representation of the PPG signal, for which motion artifacts have been removed or reduced. The time domain representation may then be used in order to extract further information of heart activity such as heart rate variability or peak-to-peak duration in heart activity.

According to an embodiment, the processing unit is further configured to: divide the cleaned time-varying frequency spectrum into sequential time segments and determine a probability distribution of a heart rate for a time segment based on the cleaned time-varying frequency spectrum within the time segment.

According to an embodiment, the time-varying frequency spectrum may include amplitude information for different frequencies. Based on such amplitude information, a likelihood that the heart rate frequency corresponds to a specific frequency may be determined and, the likelihoods may be used for determining the probability distribution of the heart rate.

By determining the probability distribution of the heart rate, the probable heart rate for a time segment may be determined. However, it has also been realized that the probability distribution may be used for further improving robustness of a PPG signal and it is therefore relevant to determine the probability distribution for motion artifact reduction.

The sequential time segments may be partially overlapping. In another embodiment, the time segments may define consecutive time intervals in the cleaned time-varying frequency spectrum.

According to an embodiment, the processing unit is further configured to determine a probability distribution of a heart rate for a current time segment based on an estimate of the heart rate of a previous time segment.

It is realized that the heart rate is not subject to very sudden changes. Rather, the heart rate of a current time segment is likely to be similar to the heart rate of a previous time segment. By determining a probability distribution of the heart rate for a current time segment based on a previous time segment, it is possible to identify indications in the PPG signal corresponding to changes in heart rate which are not natural and, hence, probably due to a motion artifact. Therefore, the determination of the probability distribution of the current time segment based on the heart rate of the previous time segment may be relevant for motion artifact reduction.

The probability distribution may be determined based on an empirically determined relationship between the heart rate of a current time segment and the heart rate of the previous time segment. The relationship may be a default relationship that is commonly used for any subject. However, the relationship may also be determined individually for a specific subject, e.g. in set-up of a system before use.

According to an embodiment, wherein the processing unit is further configured to determine, for the current time segment, a first confidence level of the probability distribution of the heart rate based on the cleaned time-varying frequency spectrum within the time segment and a second confidence level of the probability distribution of the heart rate based on the cleaned time-varying frequency spectrum within the previous time segment.

By determining confidence levels of the probability distributions, it is possible to relate the probability distributions to each other and determine how well the probability distributions may be trusted. This may be advantageously used in determining a reliable heart rate based on both the information in a current time segment and information in a previous time segment.

According to an embodiment, the processing unit is further configured to determine an estimated heart rate of the current time segment based on the probability distribution of the heart rate based on the cleaned time-varying frequency spectrum within the current time segment, the probability distribution of the heart rate based on the estimate of the heart rate of the previous time segment, the first confidence level and the second confidence level.

A determination of the estimated heart rate may thus take into account the information in a current time segment and information in a previous time segment and how well the information may be trusted. The determination of the estimated heart rate may thus ensure that residues of motion artifacts affecting the signal after the subtraction of the motion reference representation from the PPG representation may be identified and disregarded. The estimated heart rate may thus be used in further improving signal quality if a time domain representation is generated based on the cleaned signal.

According to an embodiment, the processing unit is further configured to determine whether a motion artifact situation is prevailing and, based on whether the motion artifact situation is prevailing, selectively subtract the weighted representation of the motion artifact time-frequency representation from the weighted representation of the PPG time-frequency representation.

This implies that the subtraction need only be performed when a motion artifact situation is prevailing. Thus, the processing for motion artifact reduction is only done when needed and, processing power may be saved when no motion artifact situation is prevailing. Further, the subtraction of the weighted representation of the motion artifact time-frequency representation from the weighted representation of the PPG time-frequency representation may negatively impact quality of the PPG time-frequency representation if no motion artifact situation is prevailing. Therefore, the signal quality may be better when the subtraction step is not performed if the motion artifact situation is not prevailing.

According to an embodiment, the processing unit is configured to determine whether a motion artifact situation is prevailing based on comparing at least one characteristic of the PPG signal with corresponding at least one characteristic of the motion reference signal.

This implies that determination whether a motion artifact situation is prevailing may be made based on the PPG signal and the motion reference signal. Thus, the same signals used for forming the cleaned time-varying frequency spectrum of the PPG signal may also be used for determining whether the motion artifact situation is prevailing.

However, it should be realized that a motion artifact situation may be identified using another sensor. For instance, the motion reference signal may be based on a PPG signal using a relevant wavelength, whereas the motion artifact situation may be identified using an accelerometer or another sensor specifically adapted to sense movements.

By comparing corresponding characteristics of the PPG signal and the motion reference signal, a motion artifact situation may be identified. As the PPG signal is sensitive to heart activity, whereas the motion reference signal is sensitive to movements, the relation between the signals may change depending on whether a motion artifact situation prevails or not. For instance, a power ratio between the signals may be determined and a higher power ratio between the PPG signal and the motion reference signal may indicate that no motion artifact situation prevails.

According to another embodiment, the peak-to-noise ratio of the PPG signal may be determined. A high noise level of the PPG signal may indicate a motion artifact situation, which implies that if the peak-to-noise ratio is higher, it may indicate that no motion artifact situation prevails.

It should be realized that the motion artifact situation may be identified in other ways, using the PPG signal and/or the motion reference signal and/or another signal relation to movements. The identification of the motion artifact situation may even be determined by training a machine learning algorithm to detect motion artifact situations based on any one or a combination of these signals.

According to an embodiment, the processing unit is configured to form the PPG time-frequency representation and the motion artifact time-frequency representation by calculating a continuous wavelet transform of the PPG signal and the motion reference signal, respectively.

A continuous wavelet transform (CWT) may be particularly suited for time-frequency analysis since it uses a size-adjustable window, as compared to e.g. short time Fourier transform (STFT), which has high accuracy either in time domain or frequency domain according to window size.

However, in some embodiments, STFT may anyway be used instead of CWT.

According to an embodiment, the processing unit is further configured to pre-process the received PPG signal and pre-process the received motion reference signal by bandpass filtering the PPG signal and bandpass filtering the received motion reference signal.

The heart rate of a subject is physically limited to be within a known span of frequencies. Thus, by performing bandpass filtering, noise in frequency bands which is known beforehand not to be relevant to heart activity of the subject may be removed. Thus, the preprocessing may remove any motion artifacts or other noise of uninteresting frequencies.

Heart rate of subjects may vary within a range set by 24 to 240 beats per minute. Thus, a bandpass filter may be used to pass this frequency band. According to an embodiment, the bandpass filter may pass a frequency band of 0.4-4 Hz in order to ensure that information of heart activity is retained in the preprocessed signal. It should also be realized that the bandpass filter may be dynamically controlled or adjusted before a session of acquiring PPG signals based on individual characteristics, such as age of the subject.

According to an embodiment, the system further comprises a PPG detector for generating the PPG signal and a motion detector for generating the motion reference signal. The system may thus provide detectors for acquiring the signals to be processed by the processing unit.

According to an embodiment, the PPG detector and the motion detector are arranged in a common housing. By means of arranging detectors in a common housing, the detectors may be arranged on the subject in a simple manner. The detectors may be simultaneously mounted on the subject in order to allow quickly initiating measurements of heart activity.

The detectors may be arranged in the common housing such that the motion detector is close to the PPG detector and may thus be subject to and measure the motion activity which is also affecting the PPG signal.

The processing unit may be arranged in the common housing with the PPG detector and the motion detector. This implies that the system may be arranged in a single unit and may allow acquiring signals and also process the signals for providing a cleaned time-varying frequency spectrum.

The processing unit may be further configured to analyze the cleaned time-varying frequency spectrum in order to extract information, such as heart rate and/or heart rate variability. Such extracted information may also be output to a display of the common housing, such that the subject may directly view information of heart activity on the display.

However, the processing unit may be arranged in a different unit remote from the common housing. Thus, the common housing may be provided with a communication unit, for wired or wireless communication, and may transmit, possibly pre-processed, PPG signals and motion reference signals to an external unit housing the processing unit. The communication may be over a computer network, such as the Internet, which may also allow the processing unit to be arranged on any server or computer connected to the network and may hence be said to be provided "in the cloud".

According to an embodiment, the system is a wearable device. Thus, the system may be worn by a subject allowing long-term monitoring of heart activity.

According to an embodiment, the system comprises a common housing, in which PPG detector, motion detector and processing unit may be arranged. The common housing may comprise an element allowing the housing to be worn by the subject so as to provide the system in the form of a wearable device. However, it should be realized that the system may form a wearable device in other ways, such as comprising a plurality of housings, which are each arranged to be wearable by the subject.

In one embodiment, the common housing may comprise an adhesive patch for attachments to a skin surface of the subject. The adhesive patch may provide the PPG detector and the motion detector in close relation to skin of the subject so as to enable acquiring of signals of high quality.

In other embodiments, the common housing may comprise a band or belt which may be applied around a body part, such as a wristband, for arranging the common housing on a subject. This may enable the PPG detector and the motion detector to be worn by the subject without affecting comfort of the subject.

According to an embodiment, the PPG detector may comprise a PPG light source configured for emitting green light towards a skin surface of the subject and a PPG light sensor arranged to detect reflected green light.

Using green light, a PPG signal representing a pulsatile component of blood flow may be acquired, while the PPG signal is relatively insensitive to motion artifacts as light penetrates a relatively small distance into tissue. Therefore, a PPG detector comprising a green light source and a sensor arranged to detect reflected green light may be particularly useful.

According to an embodiment, the motion detector comprises a motion reference light source configured for emitting infrared light towards a skin surface of the subject and a motion reference light sensor arranged to detect reflected infrared light.

Using infrared light, a reflectance-type PPG may be acquired which is sensitive to motion artifacts. The motion artifacts may affect the motion reference signal in a similar way as the PPG signal will be affected. Therefore, a motion detector comprising an infrared light source and a sensor arranged to detect reflected infrared light may be particularly useful.

It should be realized that the estimation of heart rate based on probability distributions from the current time segment and the previous time segment, and confidence levels of the probability distributions could be performed based on a time-frequency representation of the PPG signal, which has been obtained in any manner and has not necessarily been subject to the motion artifact reduction as defined above as the first aspect. On the contrary, motion artifact reduction may or may not have been performed and may have been performed in a different manner. The estimation of the heart rate may anyway contribute to disregarding of motion artifacts in a PPG signal.

According to a second aspect, there is provided a system for motion artifact reduction in a PPG signal representing heart activity of a subject, said system comprising: a processing unit, said processing unit being configured to: receive a PPG time-frequency representation, based on the PPG signal, divide the PPG time-frequency representation into sequential time segments and determine a probability distribution of a heart rate for a time segment based on the PPG time-frequency representation within the time segment; determine a probability distribution of a heart rate for a current time segment based on an estimate of the heart rate of a previous time segment; and determine, for the current time segment, a first confidence level of the probability distribution of the heart rate based on the cleaned time-varying frequency spectrum within the time segment and a second confidence level of the probability distribution of the heart rate based on the cleaned time-varying spectrum within the previous time segment.

Hence, by determining probability distributions from the current time segment and the previous time segment, and confidence levels of the probability distributions, it is possible to estimate a heart rate with reliability. The estimated heart rate may thus be determined in presence of motion artifacts in the PPG signal and may be used for further cleaning the signal in order to provide a cleaned time domain representation of the PPG signal.

According to a third aspect, there is provided a method for motion artifact reduction in a photoplethysmogram (PPG) signal representing heart activity of a subject, said method comprising: receiving a PPG signal carrying information of heart activity of the subject; receiving a motion reference signal carrying information of a motion of the subject; forming a PPG time-frequency representation, based on the PPG signal, and a motion reference time-frequency representation, based on the motion reference signal; and subtracting a weighted representation of the motion reference time-frequency representation from a weighted representation of the PPG time-frequency representation for reducing a motion artifact component in the PPG time-frequency representation and form a cleaned time-varying frequency spectrum of the PPG signal.

Effects and features of this third aspect are largely analogous to those described above in connection with the first aspect. Embodiments mentioned in relation to the first aspect are largely compatible with the third aspect.

According to a fourth aspect, there is provided a computer program product or a computer-readable medium storing computer-readable instructions such that when executed on a processing unit the computer program product will cause the processing unit to perform the method according to the third aspect.

Effects and features of this fourth aspect are largely analogous to those described above in connection with the first, and third aspects. Embodiments mentioned in relation to the first, and third aspects are largely compatible with the fourth aspect.

The computer-readable instructions, which may be stored in the form of a computer program product or on a computer-readable medium, may implement an algorithm for performing time domain signal reconstruction. The computer-readable instructions may thus allow a processing unit to be set up, by receiving the computer program product or the computer-readable medium, for performing the method. Hence, the computer program product or computer-readable medium may be delivered to a processing unit at any point in time in order to configure the processing unit for allowing the method to be performed.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a schematic view of a system for acquiring and processing of a PPG signal.

Fig. 2a-b are schematic view illustrating acquiring of a reflectance-type PPG signal and penetration of green and red light, respectively, in tissue.

Fig. 3 is a flow chart illustrating a method for motion artifact reduction.

Fig. 4 is a flow chart illustrating a method for time domain signal reconstruction.

Fig. 5 is a flow chart illustrating processing steps of combined motion artifact reduction and time domain signal reconstruction.

Fig. 6 is a graph illustrating a classifier for deciding between a motion or no motion situation.

Fig. 7 is a graph illustrating subtraction of a motion reference time-frequency representation from a PPG time-frequency representation.

Fig. 8a is a graph illustrating a frequency spectrum mask and Fig. 8b is a graph illustrating adjusted weights of SSA components based on the frequency spectrum mask.

Fig. 9 are graphs illustrating an original PPG signal and a reconstructed time domain signal.

Fig. 10 are graphs illustrating an original PPG signal, a motion reference signal, a reconstructed time domain signal and a de-noised signal.

## Detailed description

Ambulatory monitoring for health status/indicators of a subject requires high reliability in data collection and processing during various activities of daily life, like walking, jogging and stretching. However, during these activities, a photoplethysmogram (PPG) signal is susceptible to corruption by motion artifacts, which may originate from sensor movement, tissue deformation, and ambient light changing, etc.

Thus, if a PPG signal is to be used for long-term monitoring of heart activity of a subject, motion artifact reduction is an important issue to handle. Further, a time domain signal reconstruction of a clean, or at least relatively clean, PPG signal may allow extracting features on a short time scale, such as relating to a single heart beat or a few heart beats. This implies that heart rate variability may be extracted.

Both motion artifact reduction and time domain signal reconstruction may be applied to an acquired PPG signal. Therefore, although the present application is mainly related to motion artifact reduction, in the following description, time domain signal reconstruction will also be described. It should be realized that the time domain signal reconstruction need not necessarily be performed after the motion artifact reduction. Also, time domain signal reconstruction may be performed in other manners than those described below.

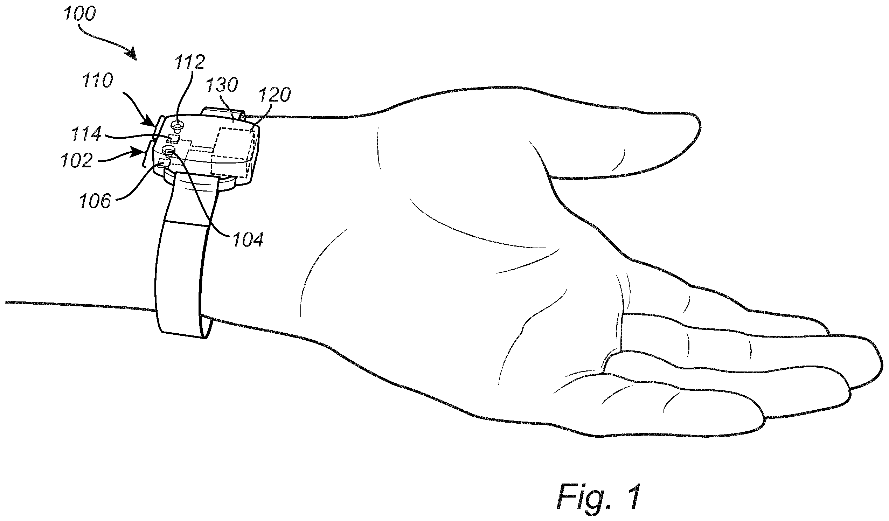

Referring now to Fig. 1, a system 100 for acquiring and processing a PPG signal will be generally described.

The system 100 may comprise a PPG detector 102. The PPG detector 102 may comprise a light source 104 and a light sensor 106 for detecting an intensity of light. The light source 104 and the light sensor 106 may be arranged on opposite sides of tissue, such as a fingertip, for acquiring an intensity of light transmitted through the tissue, so called transmittance-type PPG. However, the light source 104 and the light sensor 106 may alternatively be arranged on a common side of tissue for acquiring an intensity of light diffusely reflected by the tissue, so called reflectance-type PPG. Reflectance-type PPG may be advantageously used, because it may allow arranging the PPG detector, e.g. in a wrist-worn device, such as in a smart watch or bracelet, or in an adhesive patch which may e.g. be attached to a chest of a person.

The system 100 may further comprise a motion detector 110. The motion detector 110 may be used for acquiring a motion reference signal, which may be used for motion artifact reduction in the PPG signal acquired by the PPG detector 102.

The motion detector 110 may use any type of technology for detecting a motion artifact. The motion detector 110 may thus comprise an accelerometer or any other sensor specifically adapted to sense movements. However, as illustrated in Fig. 1, the motion detector 110 may also comprise a light source 112 and a light sensor 114 for detecting an intensity of light. The motion detector 110 may thus be configured to detect a PPG, which may be used as a motion reference signal. The motion detector 110 may use the same type of PPG measurement as used for acquiring the PPG signal, i.e. a reflectance-type PPG or transmittance-type PPG.

In one embodiment, the motion detector 110 and the PPG detector 102 may share a common light sensor 114 for detecting intensities of light based on each wavelength used by the PPG detector 102 and the motion detector 110.

The system 100 may further comprise a processing unit 120, which is configured to process the PPG signal. The processing unit 120 may be configured to process the PPG signal in order to perform motion artifact reduction and/or time domain signal reconstruction based on the acquired PPG signal. The processing performed by the processing unit 120 will be further described below.

The processing unit 120 may be arranged in a common housing 130 with the PPG detector 102 and/or the motion detector 110. This implies that the system 100 may be arranged in a single physical unit or in a few units, which may communicate with each other, through wired or wireless communication. For instance, the processing unit 120 may be provided in a common housing with one of the PPG detector 102 or the motion detector 110, whereas the other detector is provided in a separate housing and is configured to communicate an acquired signal to the processing unit 120.

The processing unit 120 may thus be arranged in a unit, which may be worn by the subject, thus allowing acquiring signals and also processing of the signals close to the subject. This may imply that the processing may be performed in real-time and analysis results may be provided directly to the subject, e.g. in a display of the housing 130.

However, the processing unit 120 may be arranged in a different unit remote from the housing 130. Thus, the housing 130 may be provided with a communication unit, for wired or wireless communication, and may transmit, possibly pre-processed, PPG signals and motion reference signals to an external unit housing the processing unit 120. The external unit may be arranged close to the subject, such as in a computer arranged in a hospital room. However, the external unit may also be very remotely placed. The communication may be over a computer network, such as the Internet, which may also allow the processing unit 120 to be arranged on any server or computer connected to the network and may hence be said to be provided "in the cloud". The processing unit 120 may then further be arranged to transmit cleaned PPG signals or extracted features back to a unit close to the subject, or to a communication unit in the housing 130, e.g. for display of results to the subject.

The processing unit 120 may be implemented in hardware, or as any combination of software and hardware. At least part of the functionality of the processing unit 120 may, for instance, be implemented as software being executed on a general-purpose computer. The system 100 may thus comprise one or more processing units, such as a central processing unit (CPU), which may execute the instructions of one or more computer programs in order to implement desired functionality.

The processing unit 120 may alternatively be implemented as firmware arranged e.g. in an embedded system, or as a specifically designed processing unit, such as an Application-Specific Integrated Circuit (ASIC) or a Field-Programmable Gate Array (FPGA).

According to an embodiment, the housing 130 may be wearable by the subject. The housing 130 may thus comprise an adhesive patch for attachment to a skin surface of the subject. The housing 130 may alternatively comprise a band element or ring-shaped element for attachment around a body part. The housing 130 could for instance comprise two band parts, which may be attached to each other in an adjustable relationship for fitting the housing 130 tightly around the body part. This may be used for arranging the housing 130 around a wrist, a finger or a torso of the subject.

Wrist-worn PPG devices such as smart watches may be used during daily ambulatory activities, which leads to relatively frequent motion artifacts. Motion in daily life can change the ambient light captured by the light sensor 106, the location of the housing 130 with respect to the wrist, and a pressure between the housing 130 and skin. All of these changes may cause fluctuations in the detected PPG signals.

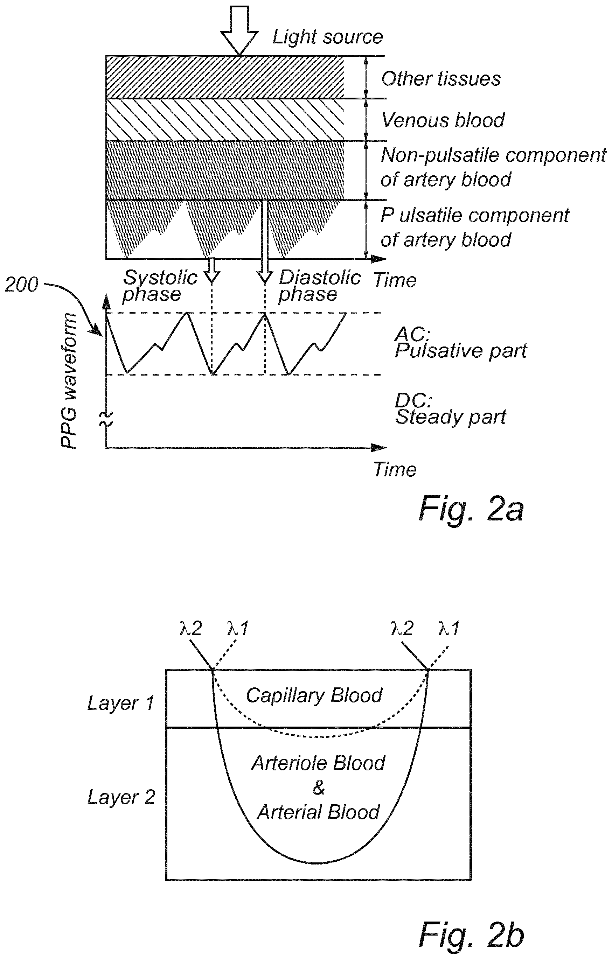

Figs 2a-b are based on figures presented in Sun Y., Thakor N., "Photoplethysmography revisited: from contact to noncontact, from point to imaging", IEEE Transactions on Biomedical Engineering, 2016, vol. 63, no. 3, pp: 463-477, and Liu J., Yan B.P.Y, Dai W.X., et al, "Multi-wavelength photoplethysmography method for skin arterial pulse extraction", Biomedical Optics Express, 2016, vol. 7, no. 10, pp: 4313-4326.

As shown in Fig. 2a, the detected PPG signal 200 can be split into two components. One component is a DC component which originates from constant absorbance by skin pigmentation, fat, muscle, bone and an average blood volume of arterial and venous blood in the illuminated tissue. The other component is an AC component which originates from the cardiac-induced variations in blood volume that are related to the cardiac rhythm (systole and diastole). The AC component may be mainly used for determining heart activity of a subject, such as for extraction of heart rate.

As illustrated in Fig. 2b, green light (indicated by a dashed line) only reaches superficial capillary bed before being reflected back to a light sensor. Red and infrared light (indicated by a solid line), however, can penetrate deeper through the skin and could reach the arteries in the subcutaneous tissue before being reflected back to a light sensor.

According to an embodiment, use is made of the fact that a PPG signal based on green light may provide a strong signal representing pulsatile component of blood, whereas a PPG signal based on infrared light may be more sensitive to motion artifacts. Thus, in one embodiment, a PPG signal may be acquired using green light, whereas a motion reference signal may be acquired using infrared light.

However, it should be realized that other wavelengths may be used. For instance, using a green or blue wavelength of light, the PPG signal is relatively insensitive to motion artifacts as light penetrates a relatively small distance into tissue. Also, using a red or infrared wavelength of light, a PPG signal representing a motion reference signal may be acquired, as the signal is relatively sensitive to motion artifacts as light penetrates a relatively large distance into tissue.

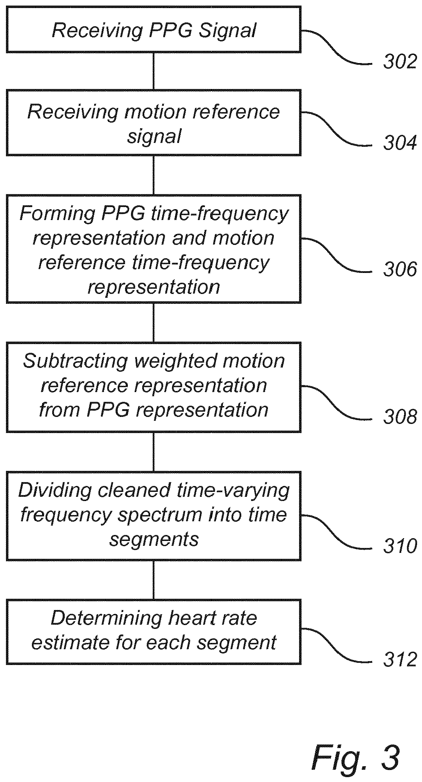

Referring now to Fig. 3, a method for motion artifact reduction will be generally described. The method may be performed by the processing unit 120.

The method comprises receiving 302 a PPG signal carrying information of heart activity of the subject. The PPG signal may be generated by the PPG detector 102 as described above. The PPG signal may or may not have been pre-processed.

The method further comprises receiving 304 a motion reference signal carrying information of a motion of the subject. The motion reference signal may be generated by the motion detector 110 as described above.

The processing unit 120 may comprise an input for receiving the PPG signal and the motion reference signal.

The input may be provided from a unit within a common housing 130, such as from sensors 106, 114 or associated circuitry in the housing 130. However, the input may alternatively be provided from a communication unit which receives the signals through wired or wireless communication with the sensors 106, 114 or associated circuitry.

The method further comprises forming 306 a PPG time-frequency representation, based on the PPG signal, and a motion reference time-frequency representation, based on the motion reference signal. Thus, the PPG signal and the motion reference signal are converted to time-frequency representations, which may allow processing a time-varying frequency information of the signals.

The method further comprises subtracting 308 a weighted representation of the motion reference time-frequency representation from a weighted representation of the PPG time-frequency representation for reducing a motion artifact component in the PPG time-frequency representation and form a cleaned time-varying frequency spectrum of the PPG signal.

The motion reference signal may be more highly influenced by the motion artifact than the PPG signal. Thus, by subtracting the motion reference signal from the PPG signal, the influence of the motion artifact on the PPG signal may be reduced without need of e.g. completely removing signal in a frequency band where a motion artifact is present, which may also imply that heart activity information of the PPG signal is eliminated. This implies that influence of motion artifacts may be reduced without information of interest, e.g. heart activity information, in the PPG signal being unnecessarily removed from the PPG signal.

The method may further comprise dividing 310 the cleaned time-varying frequency spectrum into sequential time segments. Further analysis of the PPG signal may then be performed for each of the time segments so as to allow further improving the PPG signal with regard to motion artifact reduction.

The method may further comprise determining 312 an estimate of heart rate for each time segment. The estimate may be based on probability distributions of the estimated heart rate from the current time segment and the previous time segment, and confidence levels of the probability distributions. Thus, the estimated heart rate may be determined with high reliability weighing information in a current and a previous time segment based on a confidence in the information. The estimated heart rate may be used for further cleaning the signal and may be used in determining a time domain signal reconstruction of the PPG signal.

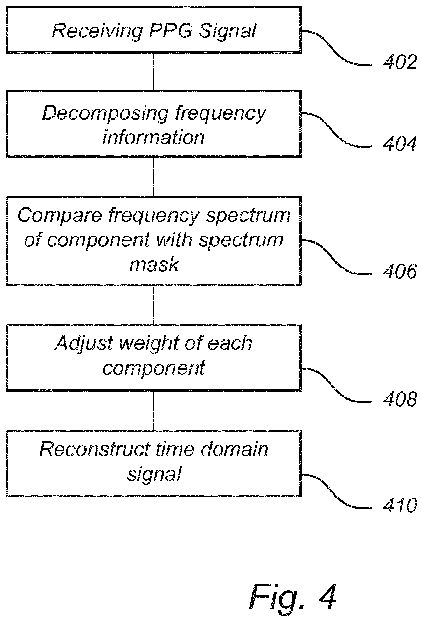

Referring now to Fig. 4, a method for time domain signal reconstruction will be generally described. The method may be performed by the processing unit 120. The method may be performed within the same processing unit, which may also be configured to perform the motion artifact reduction method as described above with regard to Fig. 3. However, it should be realized that the method may be performed as a separate process within the processing unit 120 or within a separate processing unit.

The method comprises receiving 402 a PPG signal carrying information of heart activity of the subject. The PPG signal may be a cleaned time-varying frequency spectrum signal as determined using the motion artifact reduction method described above. However, it should be realized that any type of PPG signal, in time domain, frequency domain, or time-frequency domain may be received and that the PPG signal may be a raw signal directly received from the sensor 106 or a pre-processed signal.

The method further comprises decomposing 404 frequency information of the PPG signal into a plurality of components, each component having a frequency spectrum and a weight. This implies that the frequency information may be decomposed into components having different degrees of contribution to the PPG signal. Hence, different features may be represented by different components, allowing analysis of the PPG signal. In particular, the weight of each component may indicate a magnitude of contribution of the component to the information in the PPG signal.

It should be realized that the components need not be each and every component possible to form from the information in the PPG signal. Rather, a pre-determined number of components may be formed, or only components having a weight above a threshold may be selected in forming the plurality of components based on decomposing the frequency information.

Each of the components of the plurality of components may then be further analyzed. The method thus further comprises comparing 406 the frequency spectrum of each component to a spectrum mask based on an estimate of heart rate of the subject. The spectrum mask may provide information of which frequencies are likely to represent heart activity.

The spectrum mask may be based on the estimate of the heart rate as determined in motion artifact reduction. However, the estimate of the heart rate may be formed in any other manner, even based on another type of technology for estimating heart rate.

The method further comprises adjusting 408 the weight of each component based on the comparing. Thus, the importance of the components in representing heart activity may be updated, such that components which mainly represent motion artifact frequencies or noise frequencies may be given a lower weight.

The adjusting of the weight may comprise selecting the components with highest weights after updating weights based on the comparing, such that components for which a low weight is determined the weight may be set to zero. The adjusting of the weight may also comprise applying a sigmoid function to the weights after updating the weights based on the comparing such that components having high weights may be given an even higher influence than components having low weights.

The method further comprises reconstructing 410 a time domain signal based on recombination of the plurality of components with adjusted weights. The reconstruction is thus based on updated weights of the components representing importance of the components.

This implies that a reliable and robust manner of forming a time domain signal representation of the PPG signal may be provided, while reducing influence of noise or motion artifacts in the time domain representation.

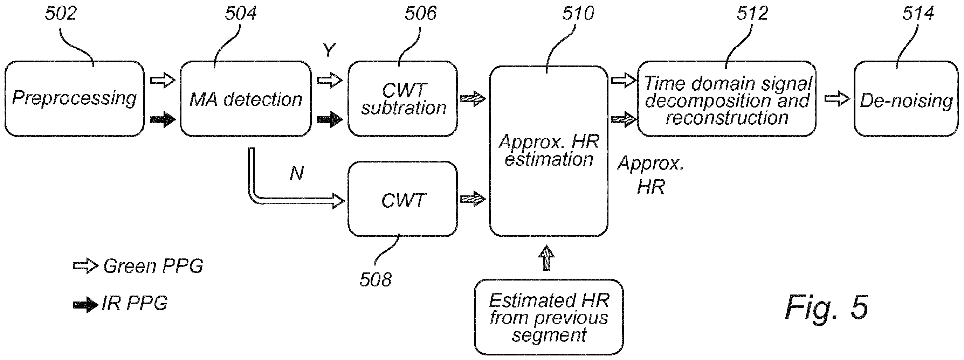

Referring now to Figs. 5-10, a detailed description of an embodiment for motion artifact reduction and time domain signal reconstruction of a PPG signal will be described. It should be realized that individual features of this embodiment may be combined in many other ways and, not necessarily with the other features described in this embodiment.

In Fig. 5, a flow chart indicating processing steps is shown. In this processing algorithm, a detected signal from an infrared (IR) PPG channel is taken as the motion reference signal, whereas a detected signal from a green PPG channel is taken as the PPG signal for heart rate (HR) extraction.

The ratio between heart activity signal information and motion artifact (MA) information is different for recorded PPG signals by green and IR light. The IR PPG signal contains more MA information and may therefore be suitable as a motion reference signal.

The frequency components of MA in recorded PPG by green and IR light are similar in periodic motion situation. The frequency components of MA in recorded PPG by green and IR light may also be similar in non-periodic motions. This implies that the IR PPG is advantageously used to represent MA content in the green PPG signal.

Further, the HR variation between 4s segments may be assumed to obey a normal distribution expressed as $X \sim N(0; 0.1)$. Thus, the HR variation $X$ is represented by a normal distribution N around 0, with a standard deviation of 0.1 Hz. This may be used as an assumption which is generally applicable to any subject. However, according to another embodiment, a distribution of the HR variation may be determined for a specific subject during set-up of the system and the determined distribution may then be used in the HR estimation, as described below.

The processing of the PPG signal and the motion reference signal starts with preprocessing 502 by bandpass filtering. Then, detection 504 of presence of motion artifact is performed based on the input PPG signal and motion reference signal. This step uses peak to average ratio from the green channel and power ratio between the green and IR PPG channels as input parameters for a classifier. A result of classification will decide whether a subtraction operation between green and IR signal will be performed or not. It should be realized that presence of MA may be determined in other ways.

If motion artifact is present, motion artifact reduction (MAR) is performed 506 by spectrum subtraction after continuous wavelet transform (CWT) of the PPG signal and the motion reference signal, resulting in a time-varying signal spectrum. In case of no motion artifact, the CWT of the green PPG signal is determined 508 and will form the time-varying signal spectrum.

After that, the HR is estimated 510 taking into consideration both the HR from a previous time segment and the spectrum from a current time segment. Approximate entropy and peak to average ratio may be used to decide a confidence level of the HR estimate for the previous time segment and the HR estimate for the current segment. Thus, the HR estimation may provide frequency tracking of the HR.

Then, time domain signal decomposition and reconstruction may be performed 512. Here, a frequency mask based on the HR estimated from the HR estimation 510 is obtained for signal reconstruction. This frequency mask is then compared with each component after singular spectrum analysis (SSA) decomposition of the PPG signal to decide the weights of the components. The PPG signal can be reconstructed by combining those components.

Finally, de-noising 514 may be applied by time domain processing. The above-described steps are discussed in more detail below.

In preprocessing 502 of the signals, to cancel an effect of light intensity, the signal is divided by its DC component that is proportional to the light intensity. The DC component may be calculated by means of recorded PPG signals of green and IR light during a stationary situation.

HR of subjects may vary within a range set by 24 to 240 beats per minute. In preprocessing 502 of the signals, both the green and IR signals are filtered with a second order infinite impulse response (IIR) bandpass filter (0.4-4Hz) to remove the noise and MA components outside a frequency band of interest. After the preprocessing, only in-band MA will remain together with the PPG signal.

The detection 504 of presence of MA may be useful to avoid subtraction of the motion reference signal from the PPG signal when no MA is present.

When no MA is present, the main components in both green and IR PPG signals are clean PPG signals. The subtraction in MAR 506 may therefore lead to cancelling the HR component itself. Therefore, in situations of no or small MA, the CWT result of the green channel (without MAR by subtracting the IR from it) may be taken to form the time-varying signal spectrum, providing HR information of the current time segment.

The motion detection may be implemented by a support vector machine (SVM), based on two parameters:

1. The peak to noise ratio of green PPG signal in frequency domain.
2. The power ratio between green PPG to IR PPG signals.

The peak to noise ratio of green PPG signal is much higher in situations of no or small MA or in periodical motion situations than in other motion situations. This is because in the first two situations, the PPG signal has a clear HR component while in the latter, the MA component can spread over frequency spectra, leading to an increase of noise.

The power ratio between green PPG to IR PPG is another indicator. Usually in no MA situation, the signal from green PPG channel (AC/DC) may be more than 10 times higher than the signal from IR PPG channel. While in motion situations, the MA component in IR may be much larger than the signal in green channel.

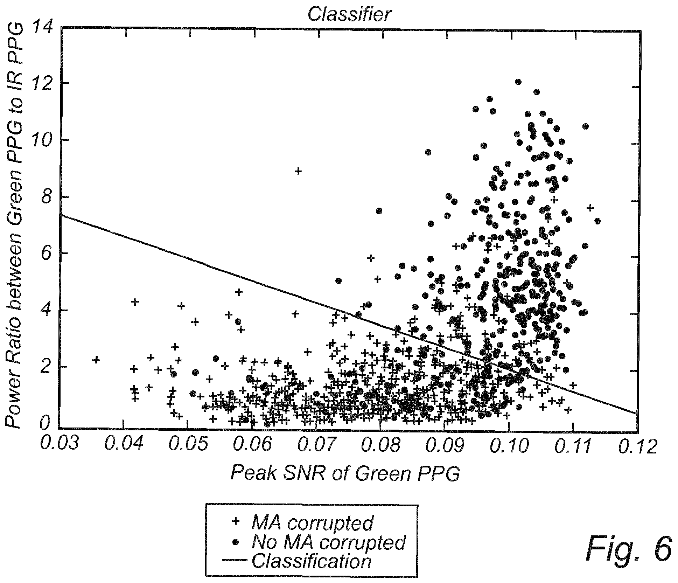

Therefore, a higher peak to noise ratio and a higher power ratio between green and IR signals tend to give a no motion decision while lower value of both parameters tend to give a motion decision. An example of a separation of the two classes by the classifier is shown in Fig. 6 for a dataset of three subjects.

The MA in IR PPG may have good frequency similarity with the MA in green PPG in periodic and non-periodic motion situations. Moreover, the ratio of MA to PPG signal in IR PPG is much higher than in green PPG. Therefore, if a scaled IR signal is subtracted from the green signal in frequency domain, the MA component can be effectively removed and the HR component will be enhanced.

PPG signal is a quasi-periodical signal. Therefore, a signal transformation having both frequency and time domain information is advantageous. Thus, a continuous wavelet transform (CWT) of the PPG signal and the motion reference signal may be used. Compared to short time Fourier transform (STFT), which has high accuracy either in time domain or frequency domain according to the window size, CWT may be better for time-frequency analysis since it uses the size-adjustable window. However, it should be realized that, in some embodiments, STFT may be used or another transform providing a time-frequency representation may be used.

In spectrum domain, the spectrum of corrupted PPG signal $P_m(f)$ can be expressed as set out in Eq. (1) below, where $P_c(f)$ is the clean PPG signal component and $MA(f)$ is the motion artifact component:

$$P_m(f) = P_c(f) + MA(f) \hspace{3cm} \text{Eq. (1)}$$

The clean PPG signal and MA signal may be assumed to be uncorrelated. The frequency domain MA removal can be obtained as expressed in Eq. (2), where a scaling factor $W$ is decided by using an adaptive filter (e.g. a wiener filter) to obtain minimum residue motion component after removal.

$$P_c(f) = P_{cG}(f) + MA_G(f) - W * \left( P_{cIR}(f) + MA_{IR}(f) \right) \hspace{1.5cm} \text{Eq. (2)}$$

Here, $P_{cG}(f)$ is the clean PPG signal component in the green PPG and $MA_G(f)$ is the motion artifact component in the green PPG, whereas $P_{cIR}(f)$ is the clean PPG signal component in the IR PPG and $MA_{IR}(f)$ is the motion artifact component in the IR PPG. The $MA_G$ and $MA_{IR}$ components have similar frequency components and the $P_{cIR}(f)$ component is very small in comparison to $MA_{IR}(f)$. Thus, the main HR component $P_{cG}(f)$ can remain intact after motion removal by means of the subtraction in Eq. (2).

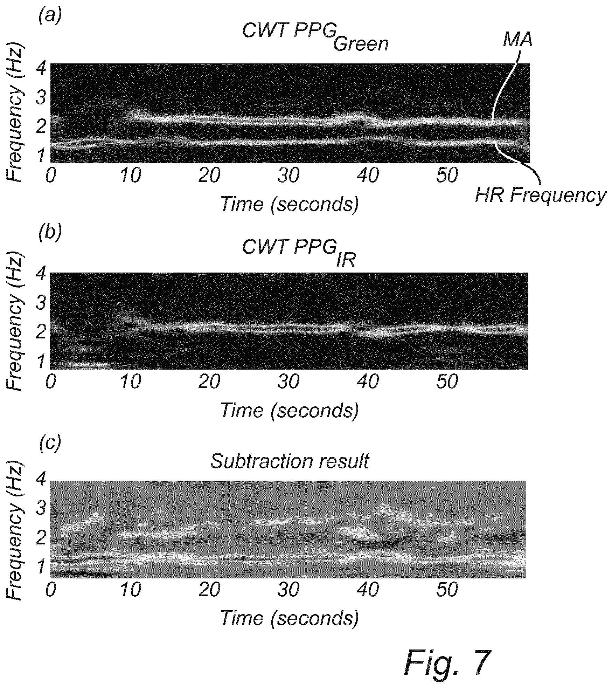

An example of periodic motion (wrist moving) removal is shown in Fig. 7 in terms of CWT representation of the signal. Comparing the time-frequency representation in graph (a) and graph (b) of Fig. 7, it can be observed that the HR component in the green PPG signal is much larger than the HR component in the IR PPG signal and the MA component in IR PPG is indeed very similar to the MA component in the green PPG. After motion removal, the MA component is cancelled and the HR component remains as shown in part (c) of Fig. 7.

However, in a no motion situation, there will be both clean PPG signals in IR and green channel, and the HR component can be cancelled by itself if the subtraction operation is performed. This is the reason why the aforementioned motion detection classifier may be advantageous. If the time segment is classified as being a no MA situation, the spectrum of green PPG signal is chosen instead of doing spectrum subtraction.

After determination of a clean time-varying signal spectrum, an estimation 510 of HR may be performed and used for improving the acquired PPG signal. The approximate HR of the current time segment is estimated based on both the time-varying signal spectrum of the current time segment and the HR frequency estimated in the previous time segment. Further, confidence levels of both the approximate HR of the current time segment and the approximate HR of the previous time segment are also used for the HR frequency decision.

The output of a time-varying signal spectrum is a sequence of frequency spectra, each corresponding to a time segment. The time segment may for instance be a 4s time segment. The frequency spectrum for a time segment may comprise only the HR related components of PPG, thanks to reduction of motion artifacts from the signal.

The amplitude information of the frequency spectrum is used to obtain a probability distribution of HR of a current time segment. There may, for instance, be 256 components in total in the frequency spectrum (from 0.5Hz to 4Hz). The number of components depend on the resolution used in determination of the time-frequency representation of the PPG signal.

The sum of the components in the frequency spectrum may be normalized to 1 by scaling each component with the same factor. This implies that a probability distribution of HR of the current time segment is given by the normalized amplitude information of the frequency spectrum.

The HR frequency estimated in the previous time segment may also be used to predict the HR in the current segment. The HR frequency for the current time segment may be based on the HR frequency of the previous time segment in combination with a probability of HR variation. Thus, the HR frequency of the current time segment may be determined as a normal distribution of $X \sim N(HR_{est}; 0.1)$. Thus, the probability distribution of HR frequency of the current time segment may be given as a normal distribution around the HR frequency of the previous time segment, $HR_{est}$, with a standard deviation of 0.1 Hz. Thus, two probability distribution functions for HR frequency estimation are available.

The confidence levels of these two distribution functions may, in one embodiment, be decided by two parameters: the approximate entropy of the signal and the peak-to-noise ratio calculated in frequency domain.

The approximate entropy may quantify the amount of regularity and the random fluctuations in time domain signals. When the signal contains only clean PPG signal with high regularity, a low entropy can be obtained. While signals with more non-periodical MA components will show a higher entropy. Therefore, the signal segments with a low entropy and a high peak-to-noise ratio can be recognized as segments which have high confidence level and thus give a true or reliable HR frequency estimation.

Thus, the two probabilities: $P(Pre_{fj} == True)$ for the estimated HR at frequency $f_j$ from previous time segment $Pre_{fj}$ and $P(Spec == True)$ for the HR distribution obtained from the current time segment $Spec$ are available. These two probabilities may be assumed to be independent.

In Bayesian inference, it is desired to determine the probability that a hypothesis HR frequency $fi$ of the current time segment $Cur_{fi}$ is true given the existence of evidence $Pre_{fj}$ (the HR frequency estimated from the previous segment) and $Spec$ (the HR frequency estimated from the spectrum of current segment) as expressed in Eq. (3).

In Eq. (3), the probability $P(Cur_{fi}|Pre_{fj} == True)$ obeys normal distribution with mean of $f_j$, and the probability $P(Cur_{fi}|Spec == True)$ is decided by its spectrum (result of CWT) as discussed above. Further, the probability $P(Cur_{fi}|Pre_{fj} == False)$ and the probability $P(Cur_{fi}|Spec == False)$ is equal to $1/m$, where $m$ is the number of frequency components or candidates fi in the frequency spectrum ($m=256$ in the present embodiment). Together with the probabilities $P(Pre_{fj} == True)$, $P(Spec == True)$, $P(Pre_{fj} == False)$, and $P(Spec == False)$, a HR frequency with a highest probability may be obtained. This approximately estimated HR frequency may then be used in signal reconstruction in order to remove MA from signal reconstruction. Also, the approximately estimated HR frequency may be used as a reliable estimate of the HR of the current time segment.

$$
\begin{aligned}
P\big(Cur_{fi}\big|Pre_{fj}, Spec\big) &= \frac{P\big(Pre_{fj}, Spec, Cur_{fi}\big)}{P\big(Spec, Cur_{fi}\big)} \\
&\propto P\big(Pre_{fj} == True, Spec == True, Cur_{fi}\big) + \\
&\quad P\big(Pre_{fj} == True, Spec == False, Cur_{fi}\big) + \qquad \text{Eq. (3)} \\
&\quad P\big(Pre_{fj} == False, Spec == True, Cur_{fi}\big) + \\
&\quad P\big(Pre_{fj} == False, Spec == False, Cur_{fi}\big)
\end{aligned}
$$

It may be noted that the resulting approximate HR can be estimated mainly from the previous time segment when the confidence level of the current time segment is low, probably because the MAR is not working well. Likewise, the HR can also be estimated mainly from the current time segment when the confidence level of the previous time segment is low. This may occur when a motion stops and the current time segment is recognized as a no MA situation or the frequency spectrum of the current time segment is more regular than the frequency spectrum of the previous time segment and, thus, the current time segment has a high confidence level.

The estimation of HR frequency improves a robustness of determination of heart activity and may also be used in improving a time domain signal reconstruction, as will now be described.

The HR usually shows small variations from beat to beat. Using HR detection in the frequency domain provides only an average HR within a segment. Analysis in the time domain could provide the peak positions that can be used to obtain beat to beat HR. Thus, reconstruction of the time domain PPG signal may allow further analysis of the heart activity.

There are two steps in the time domain signal reconstruction described below. Firstly, the time domain signal of a single time segment is decomposed into a group of oscillatory and noise components by singular spectrum analysis (SSA), providing each component with its own weight.

Secondly, a frequency mask is then used to scale these weights.

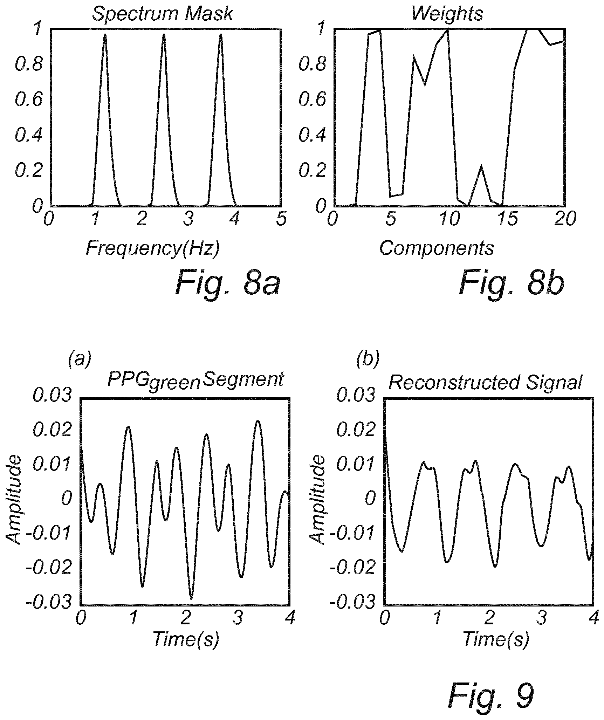

Based on the estimated approximate HR frequency, a spectrum mask can be obtained as shown in Fig. 8a. The frequency mask may be a composition of three normal distributions around the fundamental, the second and the third harmonics of the HR frequency. The normal distributions may thus provide a non-zero probability of a frequency relating to HR frequency in three ranges given by $\{f_{HR} - \Delta, f_{HR} + \Delta\}$, $\{2 * f_{HR} - \Delta, 2 * f_{HR} + \Delta\}$, and $\{3 * f_{HR} - \Delta, 3 * f_{HR} + \Delta\}$, where $\Delta$ is based on the standard deviation $\sigma = 0.1$ Hz. This distribution reflects the probability of a frequency to be part of the clean PPG signal.

The weight of each component from SSA is decided by the correlation between a frequency spectrum of the component and the frequency spectrum mask. Thus, the weight of the component, as determined above from the SSA, may be updated based on the correlation between the frequency spectrum of the component and the frequency spectrum mask. One example of resulting weights is shown in Fig. 8b, where the weight of components 1-20 from the SSA are indicated.

The updated weights may be re-scaled to a range between 0 and 1, as indicated in Fig. 8b. Also and or alternatively, weights may be scaled based on a threshold or soft threshold, such as based on a Sigmoid function, for pushing the weights towards either 0 or 1, such that mainly the components that are well correlated with the frequency spectrum mask will be taken into account in reconstruction of the time domain signal.

It should also be realized that information of motion artifacts may be used in updating of the weights. Hence, information of frequency content of motion artifacts may also be used in relation to the components of the SSA in order to lower a weight of a component that comprises frequencies determined to be related to motion artifacts. MA frequencies may be determined based on the motion reference signal, such as the IR PPG signal.

When the weights of the components of SSA have been updated, a reconstruction of a time domain signal for the time segment may be formed based on the SSA components.

The reconstruction may be performed for every time segment of 4 seconds. Fig. 9 shows one example of the original green PPG signal in part (a) and the reconstructed signal in part (b), which shows a clear HR component.

After reconstruction of the time domain signal, de-noising may be applied. The de-noising may be applied over a time period longer a time segment. Thus, for instance, the de-noising may be applied for an 8 second time period spanning two consecutive 4 second time segments.

The de-noising may operate to remove discontinuities, which may be due to the time domain signal reconstruction being based on previous steps in a 4s non-overlapping window.

The de-noising may be performed as a SSA operation to remove the noise from the signal by identifying major frequency components in the time period, allowing noise to be removed.

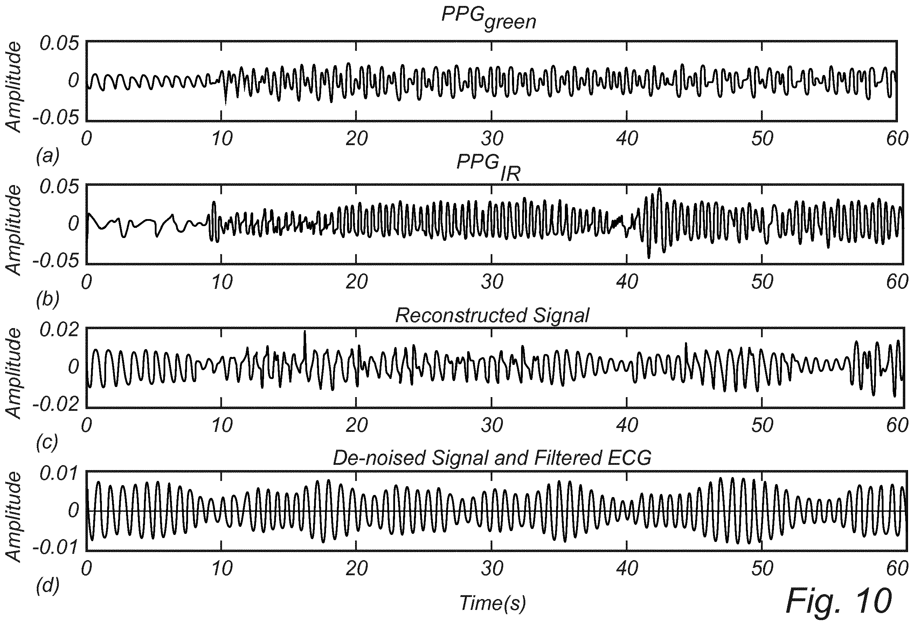

The de-noising could also apply the information from a time segment in the reconstructed time domain signal information to nearby time segments. An example is illustrated in Fig. 10, showing a green PPG signal in part (a), an IR PPG signal in part (b), a reconstructed time domain signal in part (c) and a de-noised signal in part (d). As is shown in part (c) of Fig. 10, for the period of 55-60s, the reconstruction step is not ideal, However, by means of de-noising, it is still possible to extract the clean PPG signal in this time segment with the help of previous time segment information.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, the decomposition of frequency information need not necessarily use SSA. Although SSA may be more efficient in finding relevant components, other decomposition algorithms may be used, such as independent component analysis (ICA), principle component analysis (PCA), or empirical model decomposition (EMD).

**Claims**

1. A system (100) for motion artifact reduction in a photoplethysmogram, PPG, signal representing heart activity of a subject, said system (100) comprising:

   a processing unit (120), said processing unit (120) being configured to:

   receive (302) a PPG signal carrying information of heart activity of the subject;
   receive (304) a motion reference signal carrying information of a motion of the subject;
   form (306) a PPG time-frequency representation, based on the PPG signal, and a motion reference time-frequency representation, based on the motion reference signal; and

subtract (308) a weighted representation of the motion reference time-frequency representation from a weighted representation of the PPG time-frequency representation for reducing a motion artifact component in the PPG time-frequency representation and form a cleaned time-varying frequency spectrum of the PPG signal.

2. The system according to claim 1, wherein the processing unit (120) is further configured to: divide (310) the cleaned time-varying frequency spectrum into sequential time segments and determine a probability distribution of a heart rate for a time segment based on the cleaned time-varying frequency spectrum within the time segment.

3. The system according to claim 2, wherein the processing unit (120) is further configured to determine a probability distribution of a heart rate for a current time segment based on an estimate of the heart rate of a previous time segment.

4. The system according to claim 3, wherein the processing unit (120) is further configured to determine, for the current time segment, a first confidence level of the probability distribution of the heart rate based on the cleaned time-varying frequency spectrum within the time segment and a second confidence level of the probability distribution of the heart rate based on the cleaned time-varying frequency spectrum within the previous time segment.

5. The system according to claim 4, wherein the processing unit (120) is further configured to determine (312) an estimated heart rate of the current time segment based on the probability distribution of the heart rate based on the cleaned time-varying frequency spectrum within the current time segment, the probability distribution of the heart rate based on the estimate of the heart rate of the previous time segment, the first confidence level and the second confidence level.

6. The system according to any one of the preceding claims, wherein the processing unit (120) is further configured to determine whether a motion artifact situation is prevailing and, based on whether the motion artifact situation is prevailing, selectively subtract the weighted representation of the motion artifact time-frequency representation from the weighted representation of the PPG time-frequency representation.

7. The system according to claim 6, wherein the processing unit (120) is configured to determine whether a motion artifact situation is prevailing based on comparing at least one characteristic of the PPG signal with corresponding at least one characteristic of the motion reference signal.

8. The system according to any one of the preceding claims, wherein the processing unit (120) is configured to form the PPG time-frequency representation and the motion artifact time-frequency representation by calculating a continuous wavelet transform of the PPG signal and the motion reference signal, respectively.

9. The system according to any one of the preceding claims, wherein the processing unit (120) is further configured to pre-process the received PPG signal and pre-process the received motion reference signal by bandpass filtering the PPG signal and bandpass filtering the received motion reference signal.

10. The system according to any one of the preceding claims, further comprising a PPG detector (102) for generating the PPG signal and a motion detector (110) for generating the motion reference signal.

11. The system according to any one of the preceding claims, wherein the system (100) is a wearable device.

12. The system according to any one of the preceding claims, further comprising a PPG detector (102) for generating the PPG signal, the PPG detector (102) comprising a PPG light source (104) configured for emitting green light towards a skin surface of the subject and a PPG light sensor (106) arranged to detect reflected green light.

13. The system according to any one of the preceding claims, further comprising a motion detector (110) for generating the motion reference signal, the motion detector comprising a motion reference light source (112) configured for emitting infrared light towards a skin surface of the subject and a motion reference light sensor (114) arranged to detect reflected infrared light.

14. A method for motion artifact reduction in a photoplethysmogram (PPG) signal representing heart activity of a subject, said method comprising:

receiving (302) a PPG signal carrying information of heart activity of the subject;
receiving (304) a motion reference signal carrying information of a motion of the subject;
forming (306) a PPG time-frequency representation, based on the PPG signal, and a motion reference time-frequency representation, based on the motion reference signal; and
subtracting (308) a weighted representation of the motion reference time-frequency representation from a weighted representation of the PPG time-frequency representation for reducing a motion artifact component in the PPG time-frequency representation and form a cleaned time-varying frequency spectrum of the PPG signal.

15. A computer program product or a computer-readable medium storing computer-readable instructions such that when executed on a processing unit the computer program product will cause the processing unit to perform the method according to claim 14.

Fig. 1

EP 3 501 382 A1

Fig. 2a

Fig. 2b

Receiving PPG Signal — 302

Receiving motion reference signal — 304

Forming PPG time-frequency representation and motion reference time-frequency representation — 306

Subtracting weighted motion reference representation from PPG representation — 308

Dividing cleaned time-varying frequency spectrum into time segments — 310

Determining heart rate estimate for each segment — 312

*Fig. 3*

Receiving PPG Signal — 402

Decomposing frequency information — 404

Compare frequency spectrum of component with spectrum mask — 406

Adjust weight of each component — 408

Reconstruct time domain signal — 410

Fig. 4

502 Preprocessing

504 MA detection

506 CWT subtration

508 CWT

510 Approx. HR estimation

512 Time domain signal decomposition and reconstruction

514 De-noising

Y

N

Approx. HR

Estimated HR from previous segment

Green PPG

IR PPG

*Fig. 5*

Fig. 6

(a) CWT PPG$_{Green}$

MA

HR Frequency

(b) CWT PPG$_{IR}$

(c) Subtraction result

Fig. 7

Fig. 8a

Fig. 8b

Fig. 9

PPG<sub>green</sub>

(a)

PPG<sub>IR</sub>

(b)

Reconstructed Signal

(c)

De-noised Signal and Filtered ECG

(d)

Time(s)

Fig. 10

Europäisches
Patentamt

European
Patent Office

Office européen
des brevets

# EUROPEAN SEARCH REPORT

Application Number

EP 17 21 0448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZONG CHENGZHI ET AL: "Robust heart rate estimation using wrist-based PPG signals in the presence of intense physical activities", 2015 37TH ANNUAL INTERNATIONAL CONFERENCE OF THE IEEE ENGINEERING IN MEDICINE AND BIOLOGY SOCIETY (EMBC), IEEE, 25 August 2015 (2015-08-25), pages 8078-8082, XP032812058, DOI: 10.1109/EMBC.2015.7320268 [retrieved on 2015-11-04] * page 8079, left-hand column, line 3 * * page 8080, left-hand column, paragraph 3 * * page 8081, left-hand column, paragraph 2 * * page 8081, right-hand column, paragraphs 1, 2 * * figure 2 * | 1-15 | INV. A61B5/00 A61B5/024 |
| A | US 2014/073968 A1 (ENGELBRECHT PIROW [GB] ET AL) 13 March 2014 (2014-03-13) * paragraphs [0210], [0212], [0215] - [0217], [0338] - [0340], [0367] * | 2-5,8,9 | TECHNICAL FIELDS SEARCHED (IPC) |
| A | WO 2015/180986 A1 (KONINKL PHILIPS NV [NL]) 3 December 2015 (2015-12-03) * page 19, line 11 * | 13 | A61B |
| A | WO 2016/203862 A1 (FOSTER ELECTRIC COMPANY LTD [JP]) 22 December 2016 (2016-12-22) * the whole document * | 1-15 | |

The present search report has been drawn up for all claims

| Place of search | Date of completion of the search | Examiner |
|---|---|---|
| The Hague | 4 June 2018 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 21 0448

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-2018

| Patent document cited in search report | | Publication date | Patent family member(s) | | Publication date |
|---|---|---|---|---|---|
| US 2014073968 | A1 | 13-03-2014 | NONE | | |
| WO 2015180986 | A1 | 03-12-2015 | CN | 106413530 A | 15-02-2017 |
| | | | EP | 3148404 A1 | 05-04-2017 |
| | | | JP | 2017519548 A | 20-07-2017 |
| | | | US | 2017071547 A1 | 16-03-2017 |
| | | | WO | 2015180986 A1 | 03-12-2015 |
| WO 2016203862 | A1 | 22-12-2016 | JP | 2017000625 A | 05-01-2017 |
| | | | WO | 2016203862 A1 | 22-12-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SUN Y. ; THAKOR N.** Photoplethysmography revisited: from contact to noncontact, from point to imaging. *IEEE Transactions on Biomedical Engineering,* 2016, vol. 63 (3), 463-477

- **LIU J. ; YAN B.P.Y ; DAI W.X. et al.** Multi-wavelength photoplethysmography method for skin arterial pulse extraction. *Biomedical Optics Express,* 2016, vol. 7 (10), 4313-4326